# EUROPEAN PATENT APPLICATION

(11) **EP 4 420 606 A1**
(43) Date of publication of application: **28.08.2024**
(21) Application number: 24158840.9
(22) Date of filing: 21.02.2024
(51) Int. Cl.: A61B 5/021, A61B 5/022

(54) **DEVICE AND METHOD FOR PULSE DIAGNOSIS MEASUREMENT**

(30) Priority: 23.02.2023 US 202363447656 P
(71) Applicant: Kuo, Yu-Cheng, Taipei City 100022 (TW); Kuo, Jung-Hsi, Taipei City 100022 (TW)
(72) Inventor: Kuo, Yu-Cheng, 100022 Taipei City (TW)
(74) Representative: Reich, Jochen

(57) **Abstract**

The present invention is related to a pulse diagnosis measurement device and related method, the method including the following steps: using a processor to calculate a mean arterial pressure of an organism based on a systolic blood pressure and a diastolic blood pressure of the organism; after applying a pressure onto a pulse location of the organism and adjusting the applied pressure to a first pressure value in a pressure interval using a pulse-holding device, using a sensor to sense a blood pressure wave of the organism in a first time period, so as to generate a first pulse wave signal, wherein the pressure interval is between the diastolic blood pressure and the mean arterial pressure; and using the processor to generate a plurality of first informations of a plurality of harmonics of the blood pressure wave based on the first pulse wave signal in the first time period.

## Description

### CROSS-REFERENCE TO RELATED APPLICATION

This application claims priority of US provisional application No. 63/447,656, filed on February 23, 2023, the content of which is incorporated herein in its entirety by reference.

### BACKGROUND OF THE INVENTION

### 1. Field of the Invention

The present invention relates to pulse diagnosis of living organisms, and in particular to a pulse diagnosis measurement device and method.

### 2. The Prior Arts

Pulse diagnosis is an extremely important diagnostic method in traditional Chinese medicine, and the accuracy of pulse diagnosis depends on whether a blood pressure wave of an organism can be accurately detected. When a pulse diagnosis device or an electronic blood pressure monitor is used for measuring the blood pressure wave of the organism, a cuff and a sensor are used to touch a pulse location for performing the measurement to understand a physiological condition of the organism. However, there is no ideal solution for how to make accurate measurements.

In the process of measuring blood pressure waves, the conventional technique mainly focuses on how to measure systolic blood pressure and diastolic blood pressure. The common technique is as follows: a pressure of the cuff is increased until the pressure is greater than a systolic blood pressure to block a blood flow of an arterial, and then the pressure of the cuff is decreased until a Korotkoff sound (e.g., corresponding samples of the Korotkoff sound in an electronic blood pressure monitor) exists, such that the systolic blood pressure and a diastolic blood pressure can be obtained. This measurement method will cause interference to the arteries by blocking arterial blood flow, affecting the accuracy of the measurement. Therefore, how to appropriately perform pulse diagnosis measurement is an important issue.

### SUMMARY OF THE INVENTION

In order to achieve the purpose of effectively solving the above problems, in one embodiment of the present invention, a pulse diagnosis measurement method is provided. The method comprises the following steps: using a processor to calculate a mean arterial pressure of an organism based on a systolic blood pressure and a diastolic blood pressure of the organism; after applying a pressure onto a pulse location of the organism and adjusting the applied pressure to a first pressure value in a pressure interval using a pulse-holding device, using a sensor to sense a blood pressure wave of the organism in a first time period, so as to generate a first pulse wave signal, wherein the pressure interval is between the diastolic blood pressure and the mean arterial pressure; and using the processor to generate first information of a plurality of harmonics of the blood pressure wave based on the first pulse wave signal in the first time period.

In another embodiment of the present invention, a pulse diagnosis measurement device is provided. The device comprises: a processor configured to calculate a mean arterial pressure of an organism based on a systolic blood pressure and a diastolic blood pressure of the organism; a pulse-holding device configured to apply a pressure onto a pulse location of the organism and adjusting the applied pressure to a first pressure value in a pressure interval, wherein the pressure interval is between the diastolic blood pressure and the mean arterial pressure; and a sensor configured to sense a blood pressure wave of the organism in a first time period to generate a first pulse wave signal, wherein the processor generates first information of a plurality of harmonics of the blood pressure wave based on the first pulse wave signal in the first time period.

In order for those familiar with the art to understand the purpose, characteristics and effects of the present invention, the present invention is described in detail as follows through the following specific embodiments and the accompanying drawings.

### BRIEF DESCRIPTION OF THE DRAWINGS

FIG. 1 is a block diagram of an embodiment of a pulse diagnosis measurement device of the present invention;
FIG. 2 is a flow chart of an embodiment of a pulse diagnosis measurement method of the present invention; and
FIG. 3 is a graph showing the relationship between the pressure of the pulse-holding device and the blood pressure amplitude according to an embodiment of the present invention.

### DETAILED DESCRIPTION OF THE PREFERRED EMBODIMENT

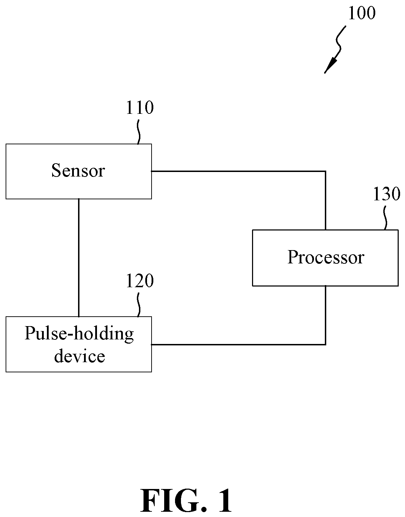

FIG. 1 is a block diagram of an embodiment of a pulse diagnosis measurement device of the present invention. The pulse diagnosis measurement device 100 may be a pulse diagnosis instrument, an electronic sphygmomanometer, a finger diagnostic device or other blood pressure wave measurement devices. As shown in FIG. 1, the pulse diagnosis measurement device 100 includes a sensor 110 for sensing a blood pressure wave of an organism (for example, a human or other animals) to generate a pulse wave signal. The pulse diagnosis measurement device 100 further includes a pulse-holding device 120 for exerting a pressure on a pulse location of the organism, so as to facilitate the sensor 110 to sense the blood pressure wave. For example, the pulse-holding device 120 may be a cuff. When the pulse location is a wrist, an arm, a finger or a neck, the cuff can enclose the pulse to apply a pressure. In one embodiment, the sensor 110 is a pressure sensor, and the pulse-holding device 120 is an inflatable cuff or bag. The pulse-holding device 120 can adjust the pressure on the pulse location by inflating and deflating, and the sensor 110 is connected to the pulse-holding device 120 to sense the changes of the internal air pressure of the pulse-holding device 120 caused by sensing pulse beats. The operation of this type of pneumatic pulse-holding device is well known to those skilled in the art, and the details will not be described here.

The pulse diagnosis measurement device 100 further includes a processor 130 for controlling the pressure applied to the pulse and performing signal processing on the pulse wave signal generated by the sensor 110, such as a Fourier transform, to generate pulse diagnosis information. According to the theory of Fourier analysis or Fourier transform, any periodic wave in a time domain can be converted into harmonics (or harmonic components) in a frequency domain. Since the blood pressure wave can be regarded as a periodic wave, the processor 130 can perform Fourier transform on the pulse wave signal which is obtained by the sensor 110 sensing the blood pressure wave, so as to generate information of the harmonics of the blood pressure wave, including frequency, amplitude, phase, etc., for use in pulse diagnosis of traditional Chinese medicine. The frequencies of these harmonics are multiples of the fundamental frequency (i.e., heart rate) of the blood pressure wave. If the multiple is an integer, then the corresponding harmonic is an integer harmonic; if a fraction, then a fractional harmonic.

In the pulse diagnosis theory of traditional Chinese medicine, different harmonics of the blood pressure waves can correspond to different meridians. For example, Table 1 shows the ten n-multiple frequency integer harmonics of the human blood pressure wave (n = 1, 2, 3, 4, 5, 6, 7, 8, 9, 10, and n = 1 is the fundamental frequency harmonic) and corresponding human meridians. The information of each harmonic can display the energy status of the corresponding meridian, which has physiological and pathological significance. Therefore, the pulse diagnosis measurement device 100 generates these harmonics by measuring human blood pressure waves, which is extremely helpful for the analysis and diagnosis of traditional Chinese medicine.

**Table 1**

| N-th harmonic | |
|---|---|
| n=1 | Jueyin Liver Channel of Foot |
| n=2 | Shaoyin Kidney Channel of Foot |
| n=3 | Taiyin Spleen Channel of Foot |
| n=4 | Taiyin Lung Channel of Hand |
| n=5 | Yangming Stomach Channel of Foot |
| n=6 | Shaoyang Gallbladder Channel of Foot |
| n=7 | Taiyang Bladder Channel of Foot |
| n=8 | Yangming Large Intestine Channel of Hand |
| n=9 | Shaoyang Sanjiao Channel of Hand |
| n=10 | Taiyang Small Intestine Channel of Hand |

One of the purposes of the present invention is to appropriately measure blood pressure waves and generate more accurate information on each harmonic of the blood pressure waves, so as to improve the accuracy of pulse diagnosis in traditional Chinese medicine. To properly measure blood pressure waves to generate accurate harmonic information, the pulse-holding device 120 must have matching resonance conditions. Specifically, the pulse-holding device 120 needs to apply an appropriate amount of pressure at the pulse location in order to produce better resonance with the beating of the pulse, so that the sensor 110 can more fully sense the pressure and energy generated by the beating of the pulse. If the applied pressure is too small, resonance will not be formed; if it is too large, it will affect the arterial blood flow and cause interference, both of which will affect the accuracy of the measurement. Therefore, in one embodiment, the pulse diagnosis measurement device 100 can dynamically adjust the pressure of the pulse-holding device 120 to an appropriate pressure interval, thereby matching the blood pressure wave and its harmonics to be measured to achieve better measurement results.

In one embodiment, the pulse diagnosis measurement device 100 is used to perform the pulse diagnosis measurement method of the present invention. Firstly, the processor 130 calculates the mean arterial pressure (MAP) of the organism based on a systolic blood pressure (SBP) and a diastolic blood pressure (DBP) of the organism (MAP is defined as (SBP+2*DBP)/3)). In one embodiment, the SBP and the DBP are measured by the pulse-holding device 120 and the sensor 110. At this time, the measurement method used can be any known method. For example, a conventional measurement method of an electronic sphygmomanometer determines SBP and DBP based on sampling points corresponding to Korotkoff sounds. In another embodiment, the SBP and DBP may be provided by other measurement devices.

Next, after the pressure applied by the pulse-holding device 120 is adjusted to a first pressure value in the pressure interval between DBP and MAP, the sensor 130 senses the blood pressure wave in a first time period to generate a first pulse wave signal. Finally, the processor 130 generates a plurality of first informations of a plurality of harmonics of the blood pressure wave according to the first pulse wave signal, i.e. each harmonic has a corresponding first information. In one embodiment, in addition to the measurement in the first time period, the pressure applied by the pulse-holding device 120 is also adjusted to a second pressure value in the pressure interval between DBP and MAP in a second time period, and the blood pressure wave is sensed to generate a second pulse wave signal, wherein the second pressure value is greater than the first pressure value. Then, the processor 130 generates a plurality of second informations of the harmonics of the blood pressure wave according to the second pulse wave signal, i.e. each harmonic has a corresponding second information. Finally, the processor 130 generates a plurality of third informations of the harmonics based on the first informations and second informations of the harmonics of the blood pressure wave, i.e. each harmonic has a corresponding third information.

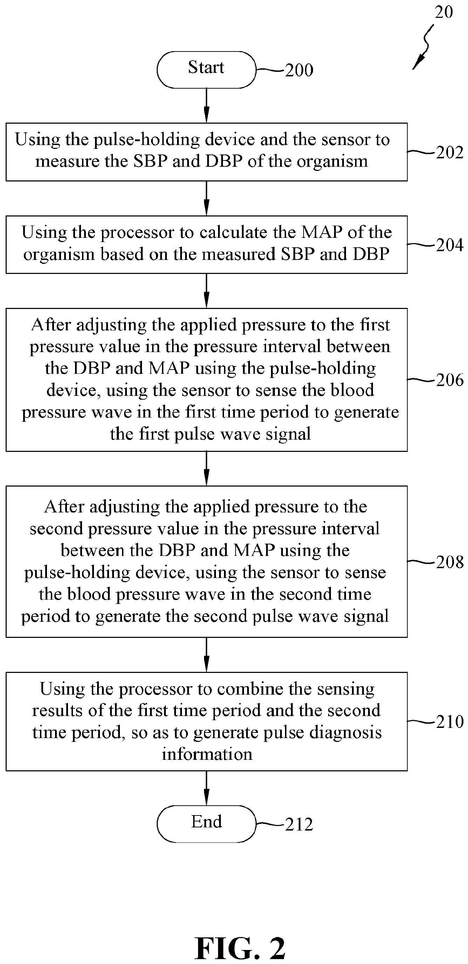

FIG. 2 is a flow chart of the pulse diagnosis measurement method 20 performed by the pulse diagnosis measurement device 100 according to an embodiment of the present invention. The pulse diagnosis measurement method 20 includes the following steps.

Step 200: Start.

Step 202: using the pulse-holding device 120 and the sensor 110 to measure the SBP and DBP of the organism.

Step 204: using the processor 130 to calculate the MAP ((SBP+2*DBP)/3) of the organism based on the measured SBP and DBP.

Step 206: After adjusting an applied pressure to a first pressure value in the pressure interval between the DBP and the MAP using the pulse-holding device 120, using the sensor 110 to sense the blood pressure wave in the first time period to generate a first pulse wave signal.

Step 208: After adjusting the applied pressure to a second pressure value in the pressure interval between the DBP and the MAP using the pulse-holding device 120, using the sensor 130 to sense the blood pressure wave in the second time period to generate a second pulse wave signal. Wherein, the second pressure value is greater than the first pressure value.

Step 210: using the processor 130 to combine the sensing results of the first time period and the second time period, so as to generate pulse diagnosis information.

Step 212: End.

In step 202, the SBP and DBP can also be provided by other measuring devices, and are not limited to those measured by the pulse-holding device 120 and the sensor 110. It has been found through research that when the pressure applied in the above-mentioned steps 206 and 208 is any pressure value in the pressure interval between the DBP and the MAP, the pulse diagnosis measurement device 100 can achieve an appropriate measurement state, so that the blood pressure wave measured in the steps 206 and 208 has appropriate resonance conditions, and will have more accurate pulse diagnosis information than the blood pressure wave generated in the previous measurement state that has not reached the pressure interval. Therefore, in step 210, the processor 130 also generates pulse diagnosis information of the organism based on the results sensed in the pressure interval, which at least includes information of the heart rate and each harmonic of blood pressure wave. The results sensed in this pressure interval will contain more accurate information, which is helpful for subsequent pulse diagnosis analysis.

It is worth mentioning that the purpose of performing two measurements with different pressure values in the pressure interval between the DBP and MAP is to improve the accuracy of harmonic measurement. Specifically, since the second pressure value is greater than the first pressure value, the blood pressure wave sensed with the first pressure value (lower pressure value) in the first time period will have better accuracy at lower frequency harmonics after performing Fourier transform thereon, and the blood pressure wave sensed with the second pressure value (higher pressure value) in the second time period will have better accuracy at higher frequency harmonics. Therefore, in step 210, when combining the sensing results in the first time period and the second time period, for example, for pulse diagnosis information related to lower frequency harmonics, the sensing results of the first time period can be used; and for pulse diagnosis information related to higher frequency harmonics, the sensing results of the second time period can be used. In this manner, the sensing results used for analysis at each harmonic will have higher accuracy.

In one embodiment, in step 210, the processor 130 generates a plurality of first informations of a plurality of harmonics of the blood pressure wave based on the first pulse wave signal in the first time period, and generates a plurality of second informations on the plurality of harmonics of the blood pressure wave based on the second pulse wave signal in the second time period. Then, the processor 130 generates a plurality of third informations of the harmonics based on the first informations and the second informations of the harmonics, wherein when the frequency of one harmonic is greater than a specific multiple of the fundamental frequency of the blood pressure wave, the second information of the harmonic is selected as the third information of the harmonic; when the frequency of one harmonic is not greater than the specific multiple of the fundamental frequency of the blood pressure wave, the first information of the harmonics is selected as the third information of the harmonic. In this way, the third information of each harmonic finally obtained will have higher accuracy. For example, the harmonics are the first to tenth harmonics, and the specific multiple is four, then for the first to fourth harmonics, the first information is selected as the third information, and for the fifth to tenth harmonics, the second information is selected as the third information.

In the above embodiment, the first pressure value is preferably the DBP, but is not limited thereto and can be any pressure value in the pressure interval between the DBP and the MAP. The second pressure value is preferably the MAP, more preferably (DBP+MAP)/2, but is not limited to this and can be any pressure value in the pressure interval between the DBP and the MAP. In addition, it should be noted that since the pressure exerted by the pulse-holding device 120 must be greater than a certain value to stably measure the blood pressure wave, if the DBP of the organism is too low (for example, a person with a DBP lower than 60 mmHg), the set first pressure value cannot be less than the specific value.

In addition, the lengths of the first time period and the second time period can be any time length that is sufficient to obtain sufficient sensing data. For example, the first time period and the second time period may have the same length, for example, 30 seconds. However, the present invention is not limited to this.

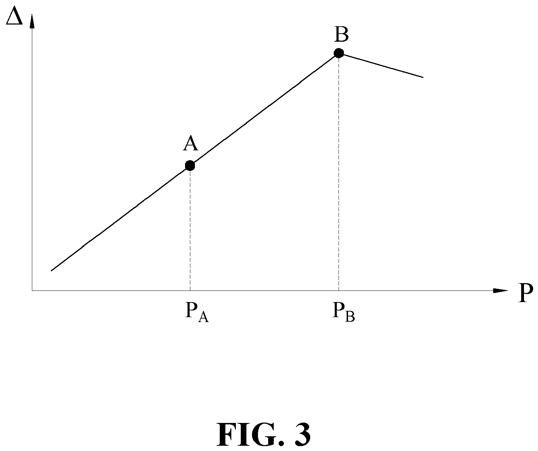

FIG. 3 is a graph showing the relationship between the pressure of the pulse-holding device 120 and the blood pressure amplitude during the adjustment of the pulse-holding device 120 in steps 206 and 208 according to an embodiment of the present invention. In FIG. 3, the horizontal axis P represents the pressure exerted by the pulse-holding device 120 and the vertical axis Δ represents the difference between the highest value and the lowest value of the blood pressure wave, that is, the blood pressure amplitude. In FIG. 3, coordinate point A is the coordinate point when the pressure applied by the pulse-holding device 120 reaches the DBP P_{A}, and coordinate point B is the coordinate point when the pressure applied by the pulse-holding device 120 reaches the MAP P_{B}. In FIG. 3, when the pulse-holding device 120 applies pressure to the pulse of the organism, as the pressure slowly increases from zero, the blood pressure amplitude will also increase. However, after the applied pressure exceeds a critical value (MAP P_{B}), the blood pressure amplitude will begin to decrease slowly. It is understandable that appropriate blood pressure amplitude can achieve better resonance effect of the pulse-holding device 120 (for example, the cuff). Therefore, it was found through experiments that the pressure interval between the DBP P_{A} and the MAP P_{B} is a better measurement pressure interval.

Referring to FIGs. 2 and 3, in step 206, the pressure applied by the pulse-holding device 120 is adjusted to the first pressure value (preferably the DBP P_{A}) in the pressure interval between the DBP P_{A} and the MAP P_{B}, so as to make the pulse diagnosis measurement device 100 reach a measurement state with appropriate resonance conditions to accurately measure the blood pressure wave and its harmonics, and the sensor 110 is used to sense the blood pressure wave in the first time period. Next, in step 208, the pressure applied by the pulse-holding device 120 is adjusted to a second pressure value (preferably the MAP P_{B}) in the pressure interval between the DBP P_{A} and the MAP P_{B}, so that the pulse diagnostic measurement device 100 reaches a measurement state with appropriate resonance conditions to accurately measure the blood pressure wave and its harmonics, and the sensor 110 is used to sense the blood pressure wave in the second time period. It can be understood that any pressure value within this interval can be used as the first pressure value and the second pressure value applied in steps 206 and 208 for measuring the blood pressure wave.

For example, Table 2 shows the SBP, DBP, and MAP of the organisms 1 to 3 measured and calculated by the pulse diagnosis measurement device 100 through the processor 130 in steps 202 and 204. Therefore, it can be known from FIG. 3 and Table 2 that: the pressure interval of the organism 1 is the interval in which the pressure exerted by the pulse-holding device 120 reaches the DBP P_{A} of 90 mmHg and then rises to the MAP P_{B} of 100 mmHg; the pressure interval of organism 2 is the interval in which the pressure exerted by the pulse-holding device 120 reaches the DBP P_{A} of 100 mmHg and then rises to the MAP P_{B} of 114 mmHg; and the pressure interval of organism 3 is the interval in which the pressure exerted by the pulse-holding device 120 reaches the DBP P_{A} of 110 mmHg and then rises to the MAP P_{B} of 128 mmHg.

**Table 2**

| | SBP | DBP | MAP |
|---|---|---|---|
| Organism 1 | 120 | 90 | 100 |
| Organism 2 | 142 | 100 | 114 |
| Organism 3 | 164 | 110 | 128 |

It can be understood that although in the above embodiment, the first pressure value and the second pressure value are selected to sense the blood pressure wave twice, the present invention is not limited thereto. Step 206 may be chosen instead of step 208 to sense the blood pressure wave only once, and the first pressure value may be chosen to be the average of DBP and MAP. In addition, more than two blood pressure wave sensing may also be performed. For example, three blood pressure wave sensing may be performed, where the first pressure value is selected as the DBP, the second pressure value is selected as the average of the DBP and the MAP, and a third pressure value (the third pressure value is greater than the second pressure value) is selected as the MAP in a third time period. Then the sensing results of the first time period, the second time period, and the third time period are combined for subsequent analysis. It is understood that those skilled in the art can adjust the selected pressure values and the number of blood pressure wave sensing as needed.

Those of ordinary skill in the art can combine, modify or change the above-described embodiments according to the spirit of the present invention, without being limited thereto. The foregoing statements, steps and/or processes (including suggested steps) can be implemented through a device. The device can be hardware, software, firmware (a combination of hardware devices and computer instructions and data, and computer instructions and data are read-only software on a hardware device), electronic system, or a combination of the above.

The above are only the preferred embodiments of the present invention. All equivalent changes and modifications made according to the patent scope of the present invention should fall within the scope of the present invention.

## Claims

1. A pulse diagnosis measurement method, comprising the following steps:
calculating, by a processor, a mean arterial pressure of an organism based on a systolic blood pressure and a diastolic blood pressure of the organism;
sensing, by a sensor, a blood pressure wave of the organism in a first time period to generate a first pulse wave signal, after applying a pressure onto a pulse location of the organism and adjusting the applied pressure to a first pressure value in a pressure interval using a pulse-holding device, wherein the pressure interval is between the diastolic blood pressure and the mean arterial pressure; and
generating, by the processor, a plurality of first informations of a plurality of harmonics of the blood pressure wave based on the first pulse wave signal in the first time period.

2. The pulse diagnosis measurement method according to claim 1, further comprising the following steps:
sensing, by the sensor, the blood pressure wave of the organism in a second time period to generate a second pulse wave signal, after adjusting the applied pressure to a second pressure value in the pressure interval using the pulse-holding device, wherein the second pressure value is greater than the first pressure value; and
generating, by the processor, a plurality of second informations of the harmonics of the blood pressure wave based on the second pulse wave signal in the second time period.

3. The pulse diagnosis measurement method according to claim 2, further comprising the following step:
generating, by the processor, a plurality of third informations of the harmonics based on the first informations and the second informations of the harmonics of the blood pressure wave.

4. The pulse diagnosis measurement method according to claim 3, wherein the step of generating the third informations of the harmonics includes:
selecting the second information of a first one of the harmonics as the third information of the first one of the harmonics when a frequency of the first one of the harmonics is greater than a specific multiple of a fundamental frequency of the blood pressure wave; and
selecting the first information of a second one of the harmonics as the third information of the second one of the harmonics when the frequency of the second one of the harmonics is not greater than the specific multiple of the fundamental frequency of the blood pressure wave.

5. The pulse diagnosis measurement method according to claim 1, wherein when the diastolic blood pressure is less than a specific value, the pressure interval is between the specific value and the mean arterial pressure.

6. The pulse diagnosis measurement method according to claim 1, wherein previous to the step of calculating the mean arterial pressure of the organism, the following step is further included:
measuring, by the pulse-holding device and the sensor, the systolic blood pressure and the diastolic blood pressure of the organism.

7. A pulse diagnosis measurement device, comprising:
a processor configured to calculate a mean arterial pressure of an organism based on a systolic blood pressure and a diastolic blood pressure of the organism;
a pulse-holding device configured to apply a pressure onto a pulse location of the organism and adjusting the applied pressure to a first pressure value in a pressure interval, wherein the pressure interval is between the diastolic blood pressure and the mean arterial pressure; and
a sensor configured to sense a blood pressure wave of the organism in a first time period to generate a first pulse wave signal,
wherein the processor generates a plurality of first informations of a plurality of harmonics of the blood pressure wave based on the first pulse wave signal in the first time period.

8. The pulse diagnosis measurement device according to claim 7, wherein after the pulse-holding device adjusts the applied pressure to a second pressure value in the pressure interval, the sensor senses the blood pressure wave in a second time period to generate a second pulse wave signal, and the processor generates a plurality of second informations of the harmonics of the blood pressure wave according to the second pulse wave signal in the second time period, wherein the second pressure value is greater than the first pressure value.

9. The pulse diagnosis measurement device according to claim 8, wherein the processor generates a plurality of third informations of the harmonics based on the first informations and the second informations of the harmonics of the blood pressure wave.

10. The pulse diagnosis measurement device according to claim 9, wherein the processor generates the third informations of the harmonics as follow:
when a frequency of a first one of the harmonics is greater than a specific multiple of a fundamental frequency of the blood pressure wave, select the second information of the first one of the harmonics as the third information of the first one of the harmonics; and
when the frequency of a second one of the harmonics is not greater than the specific multiple of the fundamental frequency of the blood pressure wave, select the first information of the second one of the harmonics as the third information of the second one of the harmonics

11. The pulse diagnosis measurement device according to claim 7, wherein when the diastolic blood pressure is less than a specific value, the pressure interval is between the specific value and the mean arterial pressure.
